# EUROPEAN PATENT APPLICATION

(11) **EP 1 767 173 A1**
(43) Date of publication of application: **28.03.2007**
(21) Application number: 06120219.8
(22) Date of filing: 06.09.2006
(51) Int. Cl.: A61F 9/007, A61B 17/32

(54) **Surgical apparatus**

(30) Priority: 21.09.2005 US 232206
(71) Applicant: Alcon, Inc., 6331 Hünenberg (CH)
(72) Inventor: Boukhny, Michael, Laguna Niguel, CA 92677 (US); Tjia, Khiun F., 8161 BM, Epe (NL)
(74) Representative: Hanna, Peter William Derek

(57) **Abstract**

A surgical apparatus (100), suitable for use in ophthalmic surgery, in particular for use in bimanual phacoemulsification, is provided. The apparatus comprises a surgical manipulation tool having a shaft (12) and a flexible irrigation sleeve (101) being generally coaxial about the manipulation tool, having a distal opening (130) through which the manipulation tool is adapted to protrude, and defining at least one irrigation port (117) adapted to allow irrigating fluid to exit the sleeve, wherein the distal opening (130) is sized and shaped to fit tightly about the shaft (12) so as to reduce or prevent irrigation fluid flow between the shaft and the irrigation sleeve.

## Description

### Background of the Invention

This invention relates generally to the field of cataract surgery and more particularly to manipulation tools and irrigation sleeves used in phacoemulsification surgery.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light that can be transmitted to the retina. This deficiency in the lens of the eye is medically known as a cataract. An accepted treatment for this condition is surgical removal of the lens and replacement of the lens function by an artificial intraocular lens (IOL).

In the United States, the majority af cataractous lenses are removed by a surgical technique called phacoemulsification. A typical surgical handpiece suitable for phacoemulsification procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

Recently, a modified phacoemulsification technique called "bimanual" phacoemulsification has been adopted by many surgeons. With the bimanual technique, the irrigation sleeve is removed from around the ultrasonically driven tip. This allows for the small tip to be inserted into the eye through a smaller incision. Irrigation fluid is supplied by a second irrigating tip. The second tip may include a manipulation tool. Additional information concerning traditional phacoemulsification and bimanual phacoemulsification is included in U.S. Patent Publication No. US 2003/0069594 A1. And in particular, Paragraphs [0036] through [0037] and FIGS. 6-8. As described in this reference, the second instrument does not use an outer silicone infusion sleeve. Rather the shaft of the manipulation tool is either hollow with irrigation ports, or solid with a separate hollow irrigating conduit containing irrigation ports. Without the outer silicone sleeve, sealing of the wound is minimal. This allows excessive irrigating fluid to escape out of the eye through the wound. Excessive wound leakage can cause shallowing of the anterior chamber, excessive turbulence and premature removal of the protective viscoelastic material. Excessive wound leakage can also cause over-hydration of the wound tissue, possibly resulting in edema.

Recently, it has been suggested that traditional one-handed phacoemulsification can be conducted through a relatively small incision by reducing the diameter of the phacoemulsification tip/sleeve. A second irrigation/aspiration tip, with or without an attached manipulation tool, may also be used to provide addition irrigation and aspiration. Such an arrangement minimizes wound leakage, thereby helping to avoid over-hydration of the wound, low intraocular pressure, excessive turbulence and premature removal of the viscoelastic material. The annular gap between the phaco tip and sleeve is used as an irrigation fluid pathway. Irrigation ports are provided on the sides of the sleeve to direct irrigating fluid out of and away from the aspiration port. This fluid flow out of the distal end of the sleeve tends to push material away from the aspiration port. In addition, the relatively unsupported distal end of the sleeve is compressed easily and pressed backward on the manipulator during insertion into the wound.

Therefore, a need continues to exist for an irrigation sleeve that seals the shaft of the manipulation tool.

### Brief Summary of the Invention

The present invention improves upon the prior art by providing a surgical apparatus having an irrigation sleeve that fits around the shaft of the manipulation tool and seals the shaft so as to reduce or prevent the flow of irrigating fluid out of the sleeve from around the shaft.

Accordingly, one objective of the present invention is to provide an irrigation sleeve that fits around the shaft of the manipulation tool and seals the shaft.

Another objective of the present invention is to provide an irrigation sleeve that reduces or prevents the flow of irrigating fluid out of the sleeve from around the shaft.

These and other advantages and objectives of the present invention will become apparent from the detailed description and claims that follow.

### Brief Description of the Drawings

FIG. 1 is a partial side elevational schematic of prior art surgical manipulation tools and sleeves.
FIG. 2 is a partial end elevational schematic view of the irrigation sleeve of the present invention.

### Detailed Description of the Invention

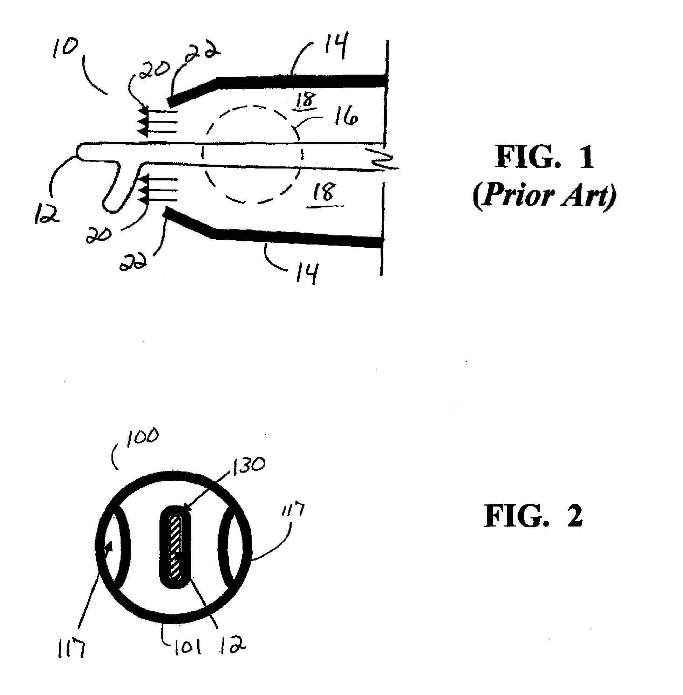

As best seen in FIG. 1, prior art devices 10 generally include relatively soft, flexible irrigation sleeve 14 coaxial about surgical manipulator 12. Manipulator 12 may be a hook or any other suitable manipulation device, such as a chopper, spatula or other desired device known in the art. Irrigation sleeve 14 contains port or ports 16 that allow irrigating fluid flowing down gap 18 between manipulator 12 and sleeve 14 to exit out of the side of sleeve 14, but because manipulator 12 is generally made to be very small in diameter, gap 18 can be relatively large and allow irrigation fluid flow 20 out of distal end 22 of sleeve 14. Unsupported distal end 22 of sleeve 14 may also be compressed, and collapse against manipulator 12 when entering a tight incision. Such compression of sleeve 14 can cause sleeve 14 to be pulled backward on manipulator 12 during insertion into the wound.

As seen in FIG. 2, apparatus 100 of the present invention includes irrigation sleeve 101 that is of a construction similar to that of prior art sleeves and contains outflow holes 117 and distal opening 130. Distal opening 130 is generally rectangular or oval and is sized and shaped to fit tightly over shaft 12. Distal opening 130 maybe preformed in sleeve 100 or formed in sleeve 100 by pushing shaft 12 through sleeve 100, thereby ensuring that sleeve 100 fits tightly around shaft 12. Such a tight fit reduces or prevents flow 20 out of sleeve 100 around shaft 12.

This description is given for purposes of illustration and explanation. It will be apparent to those skilled in the relevant art that changes and modifications may be made to the invention described above without departing from its scope

## Claims

1. A surgical apparatus (100), comprising:
a) a surgical manipulation tool having a shaft (12); and
b) a flexible irrigation sleeve (101) being generally coaxial about the manipulation tool, having a distal opening (130) through which the manipulation tool is adapted to protrude, and defining at least one irrigation port (117) adapted to allow irrigating fluid to exit the sleeve,
**characterized in that** the distal opening (130) is sized and shaped to fit tightly about the shaft (12) so as to reduce or prevent irrigation fluid flow between the shaft and the irrigation sleeve.

2. The surgical apparatus of claim 1, wherein said at least one irrigation port comprises two outflow holes (117) arranged one to each side of and separated from the distal opening (130).

3. The surgical apparatus of claim 1 or claim 2, wherein the distal opening (130) is preformed in the irrigation sleeve (101).

4. The surgical apparatus of claim 1 or claim 2, wherein the distal opening (130) is formed in the irrigation sleeve (101) by pushing the shaft (12) through the sleeve.
